# EUROPEAN PATENT APPLICATION

(11) **EP 1 712 238 A1**
(43) Date of publication of application: **18.10.2006**
(21) Application number: 05290796.1
(22) Date of filing: 11.04.2005
(51) Int. Cl.: A61K 39/00, A61K 31/704, A61P 35/00

(54) **Anthracyclin induced immunogenic dead or dying cells composition**

(71) Applicant: Institut Gustave Roussy, 94800 Villejuif (FR)
(72) Inventor: Kroemer, Guido, 92160 Antony (FR); Zitvogel, Laurence, 92160 Antony (FR); Casares, Noelia, Cité Int.Univ. Collège d'Espagne, 75014 Paris (FR); Pequignot, Marie, 94800 Villejuif (FR)
(74) Representative: Texier, Christian

(57) **Abstract**

The present invention is aimed at a composition comprising immunogenic dead or dying cancer cells or infected cells and fragments or fractions thereof obtained by treatment with anthracyclins, to a process of manufacturing said composition and to its use for treating cancer, for preparing a vaccine or for treating infectious diseases. It also relates to a medicament comprising at least one anthracyclin to be injected into or at the vicinity of the tumor.

## Description

The present invention is aimed at a composition comprising immunogenic dead or dying cancer cells or infected cells and fragments or fractions thereof obtained by treatment with anthracyclins, to a process of manufacturing said composition and to its use for treating cancer and for preparing a vaccine or for treating infectious diseases. It also relates to a medicament comprising at least one anthracyclin to be injected into or at the vicinity of the tumor.

### Background of the invention

The complete and permanent success of non-surgical cancer therapy relies on the targeting of all tumor cells (including cancer stem cells) or - theoretically - on the direct removal of a fraction of the tumor, accompanied by a "bystander effect" in which the immune system recognizes, attacks and eradicates the remaining tumor cells.

Unfortunately, however, most widely used cytotoxic anti-cancer agents exert immunosuppressive side effects. Even worse, the preponderant type of cell death induced by chemotherapy is apoptosis, and apoptosis is frequently but not unanimously viewed as immunologically silent (leading to ignorance by the immune system) or even as tolerogenic (actively down-regulating the specific anti-tumour immune response)^{1,2}. Thus, even after an initial therapeutic success, patients typically fail to mount a clinically relevant anti-tumor immune response and eventually succumb to tumor cell variants that escape from chemotherapy.

Although apoptosis is itself non-uniform with respect to the programmed signaling events responsible for cell death³, it is morphologically defined as a uniform type of cell death accompanied by chromatin condensation (pyknosis) and nuclear fragmentation (karyorhexis), occurring within an intact plasma membrane⁴. It thus differs from necrosis in which the plasma membrane is destroyed early during the death process. In biochemical terms, apoptosis is frequently accompanied by the activation of a specific subset of cysteine kinases, the caspases³. Since apoptosis is a physiological process that attains several millions of cells per second in the human body, it is inferred that this type of cell death occurs without emitting "danger signals" that would elicit a productive immune responses. Apoptotic cells would hide away from immune recognition, because they would be rapidly recognized and silently phagocytosed in an efficient manner. Thus, defects in the recognition/phagocytosis of apoptotic cells breach autotolerance and trigger autoimmune reactions⁶⁻⁸. Moreover, apoptotic cells would mediate active immunosuppression by inhibiting the production of immunostimulatory cytokines (e.g. interleukin-1ß by macrophages and interleukin-12 by dendritic cells, DC)^{9,10}, by stimulating the production of immunosuppressive factors (e.g. transforming growth factor)¹¹, and/or by eliciting antigen-specific immune tolerance¹²⁻¹⁴. In apparent contrast with this notion, however, DC can capture apoptotic tumor cells in vitro and cross-present antigen derived from internalized dying cells on MHC class I molecules for recognition by CD8⁺ T cells in vivo, thus eliciting a productive immune response^{2,15-20}, including in clinical trials²¹. Systematic comparisons of the immunogenicity of apoptotic versus necrotic tumor cells have been inconclusive²²⁻²⁵, however, suggesting that the nature of tumor cells and/or that the death-inducing stimulus would influence the experimental outcome¹.

Driven by these uncertainties and incognita, we decided to explore the possibility of inducing immunogenic tumor cell apoptosis by chemotherapy. Here, we show that anthracyclins are capable of eliciting immunogenic cell death in vitro, ex vivo and in vivo, in a fashion that is rigorously caspase-dependent.

These results have profound implications for the design of chemotherapeutic regimens with immunogenic properties and allow for the first time to prepare a tumor immunogenic composition useful as a vaccine by the induction of immunogenic cell death of tumor cells of a patient. This discovery is applicable to infectious diseases as well.

### Description

Therefore, in a first aspect, the present invention is aimed at a process of manufacturing an immunogenic composition comprising the steps consisting of:
a) suspending or plating tumor cells or infected cells in a culture media,
b) treating said cells with at least one antineoplastic compound selected from anthracyclins and vinorelbine in a sufficient amount to induce cellular apoptosis, wherein said apoptosis induced by anthracyclins produces immunogenic dead or dying cells, and
c) recovering immunogenic dead or dying cells and fragments thereof from the culture media.

In a particular embodiment, the tumor cells in step a) are the tumor cells of a given human subject. In this regard, the process may optionally comprise the step of isolating tumor cells from a tumor biopsy or sample. Thus, in this embodiment, the process allows the manufacture of a tailored immunogenic composition from one patient's own cancer cells to treat said patient (autologous treatment).

Alternatively, the process may be performed using established cancer cell lines or freshly harvested cancer cells from one or several patients. For example, the process allows the production of an immunogenic composition to treat breast cancer from one or several breast cancer cell lines treated as explicited above with at least one anthracyclin to obtain immunogenic breast cancer dead or dying cells and fragments thereof (allogenic treatment). This process may be applied to solid tumors such as carcinomas, melanomas and sarcomas including but not limited to breast, lung, prostate or skin cancer but also to blood cancer such as lymphomas and leukemias.

Alternatively, cells in step a) are infected cells including cells infected with a virus such as HIV or Hepatitis C virus.

The above process of manufacturing may further comprise one or several of the following steps : purification, elutriation, ultracentrifugation and loading into myeloid or plasmacytoid dendritic cells of immunogenic dead or dying cells and fragments thereof.

The process as defiend above allows to target specifically in vivo appropriate antigen presenting cells such as plasmacytoid dendritic cells or myeloid dendritic cells for optimal CTL priming or T cell activation. It also allows ex vivo pulsing or loading of tumor antigens on to ex vivo propagated allogeneic or autologous dendritic cells for further adoptive transfer in vivo in tumor bearing patients or infected patients.

In the above process, the immunogenic composition is useful as a medicament or a vaccine for preventing or treating cancer.

By anthracyclin compounds, it will be understood that the invention embraces any compounds known in the art to have identical core structure and presenting antineoplastic activity, for example any anthracyclin glycosides, such as doxorubicin (US 3,590,028), 4'-epi-doxorubicin (i.e. epirubicin, US 4,039,663), 3',4'-diepidaunorubicin and 3',4'-diepidoxorubicin (US 4,112,076), 4'-desoxy-doxorubicin (i.e. esorubicin), daunorubicin, 5-iminodaunorubicin and 4-demethoxy-daunorubicin (i.e. idarubicin).
Formula of these compounds is shown below :

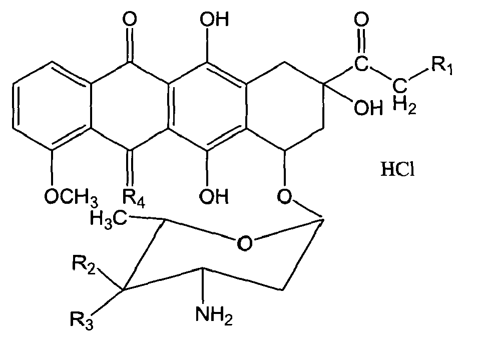

| Wherein | **R1** | **R2** | **R3** | **R4** |
|---|---|---|---|---|
| Doxorubicin | OH | H | OH | O |
| Daunorubicin | H | H | OH | O |
| Epirubicin | OH | OH | H | O |
| 5-iminodaunorubicin | H | H | OH | NH |

In the above formula, the invention also embraces the compounds wherein R1, R2, R3 are independently selected from H, OH, a halogen, a C1-C4 alkyl, alkenyl, or alkoxy group.

In a preferred embodiment, cancer cells are treated with doxorubicin (4-(4-amino-3-hydroxy-2-methyl-tetrahydropyran-6-yl)oxy-2,5,12-trihydroxy-2-(2-hydroxyacetyl)-7-methoxy-1,2,3,4-tetrahydrotetracene-6,11-dione - CAS 25316-40-9).

The invention may also be practiced with the antineoplastic compound vinorelbine (CAS Registry Number 71486-22-1) of formula :

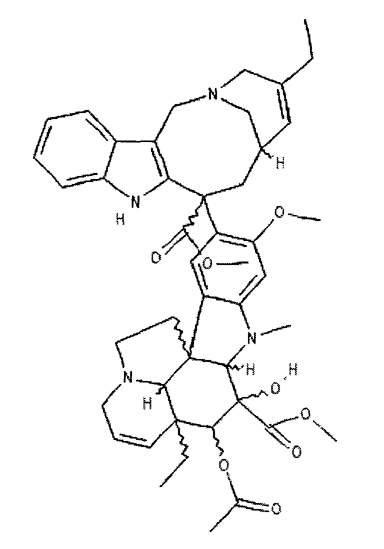

This process is useful for the manufacture of a medicament or a vaccine for preventing or treating cancer as well as infectious diseases, such as viral infection including but not limited to HIV and Hepatitis C infection.

In a second aspect, the invention relates to an immunogenic composition comprising immunogenic dead or dying cells or infected dead or dying cells and fragments thereof obtainable by the process as defined above. In fact, it relates to a composition comprising immunogenic dead or dying cancer cells and fragments or fractions thereof obtained by treatment with anthracyclins. It also relates to a composition comprising immunogenic dead or dying infected cells and fragments or fractions thereof obtained by treatment with anthracyclins.

Preferably, this composition comprises anthracyclin glycosides treated cancer cells or infected cells and fragments thereof which can be in the form of a dead or dying cellular extract or fraction thereof or of a purified cellular lysate. The composition may further comprise one of the following : a pharmaceutical acceptable carrier, one or several adjuvant(s) and/or active ingredient(s), in particular agents which are cytotoxic for tumors, such as a compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin., vinorelbine, cytokines and interleukines.

For example, the composition may further comprise one or several of the following :
- caspase endogenous inhibitors, suh as IAP proteins,
- adjuvants such as interferon-alpha (IFN-α), granulocyte-macrophage colony stimulating factor, soluble CD40 ligand, Flt3 ligand and/or interleukin-2 (IL-2),
- tyrosine kinase inhibitors such as c-kit inhibitors,
- ligands of Toll-like receptors,
- neutralizing antibodies such as anti-CD25, anti-CTLA4,
- stimulating antibodies such as anti-CD40,
- cyclophosphamides in an amount suitable for immunostimulation,
- small interfering RNAs (SiRNA) inhibiting immunosupressive genes.
Thus, herein, the composition may be in the form of a combined composition for simultaneous or separate administration.

For example, it may comprise a pharmaceutical vehicle selected from positively or negatively charged liposomes, nanoparticles or lipid emulsions.

It is preferably formulated to be suitable for intravenous, intradermic, intramuscular or subcutaneous injection, as well as oral or nasal administration.

In a third aspect, the invention concerns an extract of immunogenic dead or dying cancer cells or infected cells and fragments thereof obtainable by the process as defined above, especially in the form of a dead or dying cellular extract or fraction thereof or of a purified cellular lysate.

The invention also relates to the use of an extract of immunogenic dead or dying cancer cells or infected cells and fragments thereof for preparing a medicament for preventing or treating cancer or infecious diseases. It will be understood that the invention may apply to human as well as to other mammals, such as domestic pets and also cattle.

The invention is also directed to a method of preventing or treating cancer or infectious diseases comprising administering a composition or extract of immunogenic dead or dying cancer cells as depicted above in a patient in need of such treatment. More particularly, this method includes :
a) obtaining fresh cancer cells or infected cells from a patient,
b) suspending or plating said cells a) in a culture media,
c) treating cells of step b) with at least one antineoplastic compound selected from anthracyclins in a sufficient amount to induce cellular apoptosis, wherein said apoptosis induced by anthracyclins produces immunogenic tumor dead or dying cells,
d) recovering immunogenic tumor dead or dying cells and fragments thereof from the culture media, and
e) Administering the immunogenic dead or dying cells and fragments thereof, optionally purified, to said patient.

As mentioned above, step d) may further comprise extraction, filtration, purification, sterilization protocols to obtain an administrable composition. The preparation may also be lyophilized and then resuspended in a solution or buffer such as an appropriate sterile saline solution. In this configuration, one or several administrations over time may be applied to obtained optimized immune response against cancer.
The above method is tailored to one particular patient since the immunogenic dead or dying cells and fragments thereof composition is prepared from the cancer cells or infected cells of said patient.

Nevertheless, it shall be understood, the invention may be practiced using one or several cell lines corresponding to one particular cancer type, for example breast carcinoma. Here, the invention is directed to a method of preventing or treating cancer comprising administering a composition or extract of immunogenic dead or dying cancer cells as depicted above in a patient in need of such treatment comprising :
a) suspending or plating in a culture media cancer cells from cancer cell lines corresponding to the cancer type afflicting said patient,
b) treating cells of step a) with at least one antineoplastic compound selected from anthracyclins in a sufficient amount to induce cellular apoptosis, wherein said apoptosis induced by anthracyclins leads to immunogenic tumor cell death,
d) recovering immunogenic dead or dying cells and fragments thereof from the culture media, and
e) administering the immunogenic dead or dying cells and fragments thereof, optionally purified, to said patient.

In this embodiment, the invention offers a method for preparing and administering different immunogenic compositions that are each specific to one cancer type.

The cells of step a) may derive from established appropriate cell lines or from harvested cancer cells from one or several patients afflicted with a particular cancer including solid tumors such as carcinomas, melanomas and sarcomas including but not limited to breast, lung, prostate or skin cancer but also to blood cancer such as lymphomas and leukemias.

In the above methods, the immunogenic composition or cell extract administered may also comprise at least one antineoplastic compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin. In this regard, this composition of the invention may advantageously be injected directly in situ into the cancer mass or at the vicinity of localized cancer cells of the patient.

In still another embodiment, the invention is aimed at a method of preventing or treating cancer comprising injected directly in situ into the cancer mass or at the vicinity of localized cancer cells of the patient a compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin in an amount sufficient to induce immunogenic cancer cell death.

In other words, the invention relates to the use of a compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin for preparing a medicament suitable to direct injection in situ into the cancer mass or at the vicinity of localized cancer cells of the patient for inducing immunogenic cancer cell death. This medicament is applicable to the treatment of solid tumors such as carcinomas, melanomas and sarcomas including but not limited to breast, lung, prostate or skin cancer but also to blood cancer such as lymphomas and leukemias.

It is important to underscore the fact that the immunogenic effects of anthracyclin-treated tumor cells were observed in the absence of any adjuvant or co-stimulus. Hence, co-application of adjuvants may be provided to further improve the efficacy of the vaccination schedule. As a fascinating possibility, our invention proposes a strategy of inducing immunogenic cell death in established tumors, either ex vivo or in vivo.

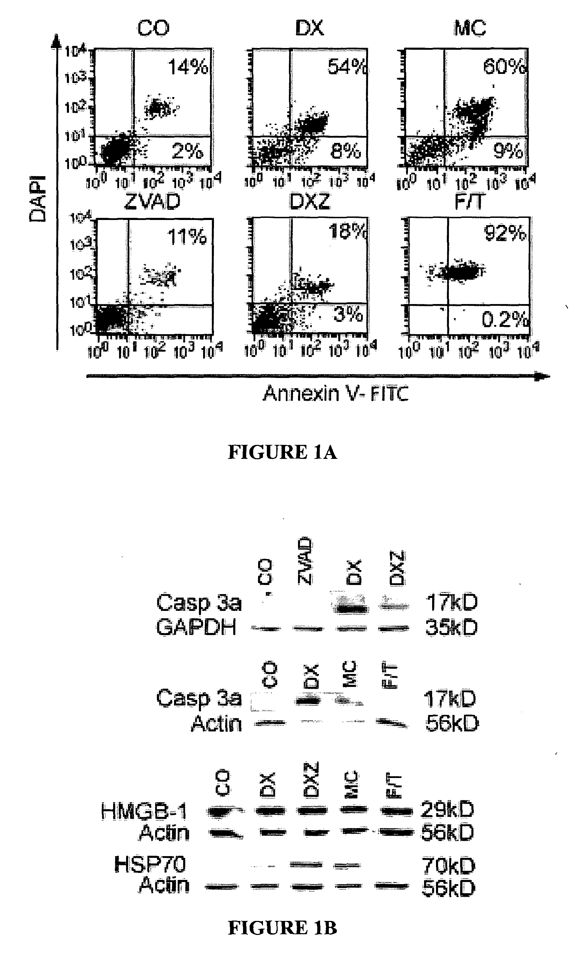

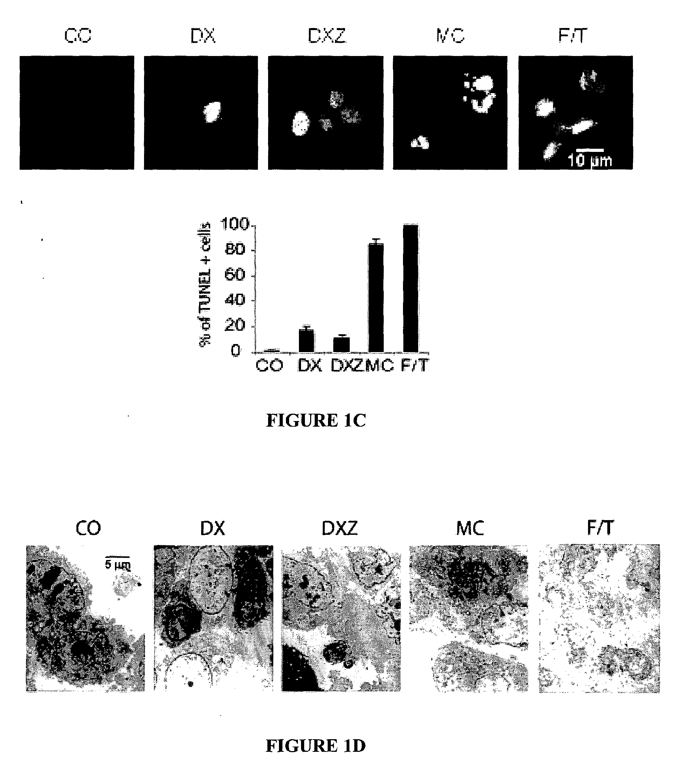

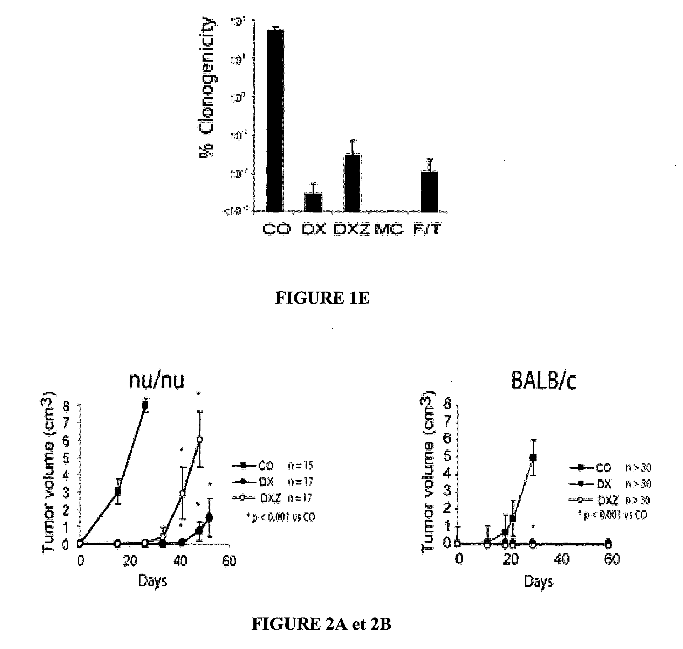

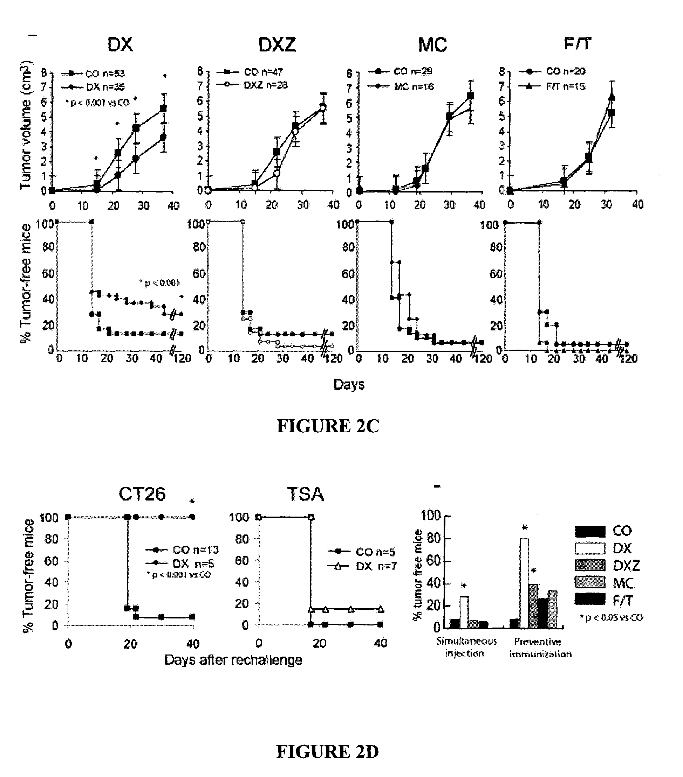

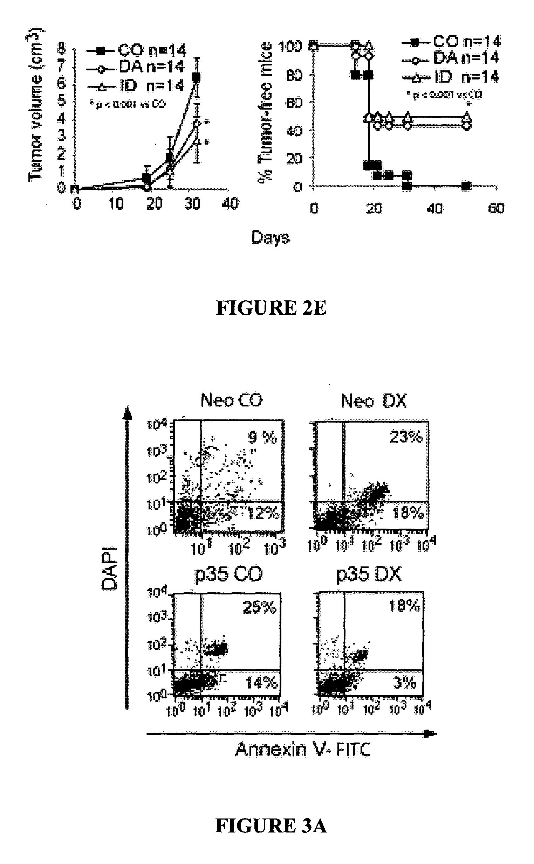

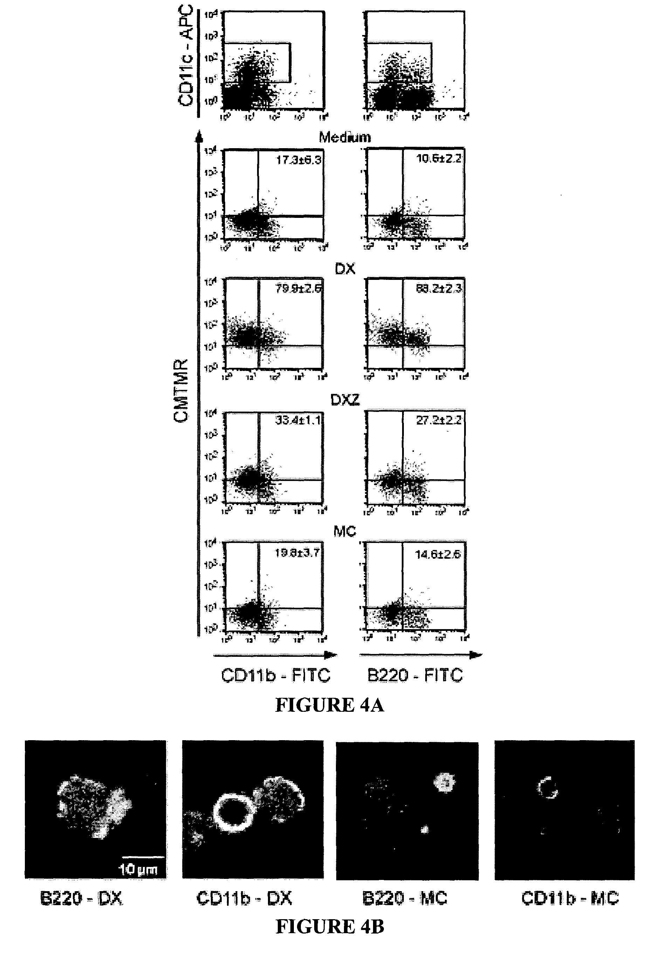

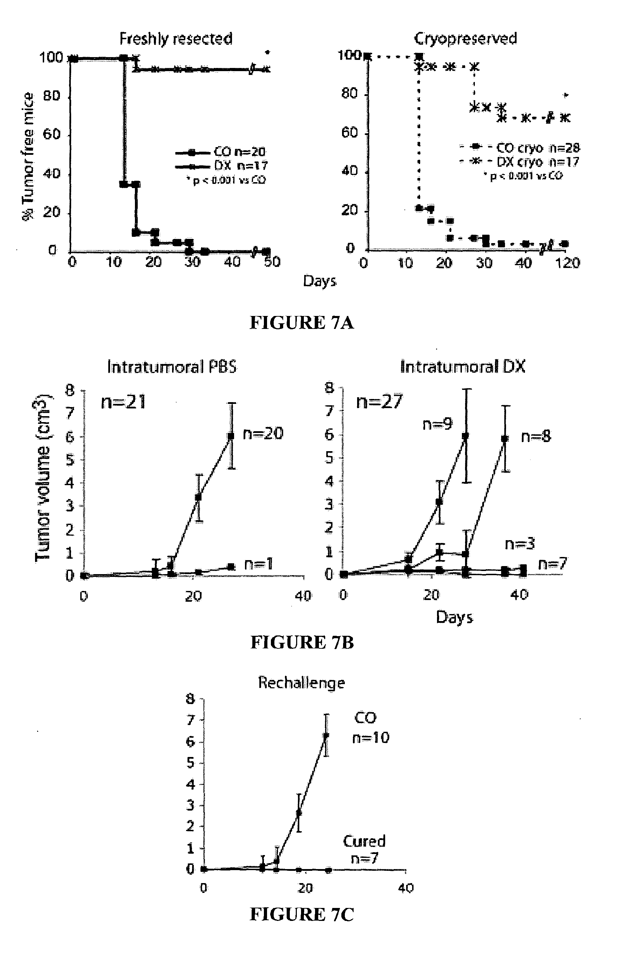

Even though apoptosis is often considered as an immunologically silent or even tolerogenic death modality^{6-14,33}, we challenge here this viewpoint and we present data demonstrating that anthracyclins-induced apoptosis can elicit an effective anti-tumor immune response. While anthracyclins (and in particular DX, which we used as a model agent) elicited immunogenic apoptosis in vitro (Fig. 2, 3, 5, 6), ex vivo (Fig. 7A) and in vivo (Fig. 7B), MC (Fig. 2, 4C, 5A) was relatively inefficient in eliciting immunogenic cell death. Preparations of DX or MC-treated cells that contained a similar percentage of dying cells (Fig. 1a) were injected, and the morphological aspect of cell death elicited by DX and MC was similar (Fig. 1C,D). Both were accompanied by caspase activation (Fig. 1B), without any difference in the expression of some major endogenous adjuvants (such as HSP70 and HMGB-1, Fig. 1B), and the only difference resided in the degree of DNA fragmentation, which was more advanced with MC (Fig. 1C). Moreover, DC phagocytosed MC-treated cells less efficiently than DX-treated cells (Fig. 4A).

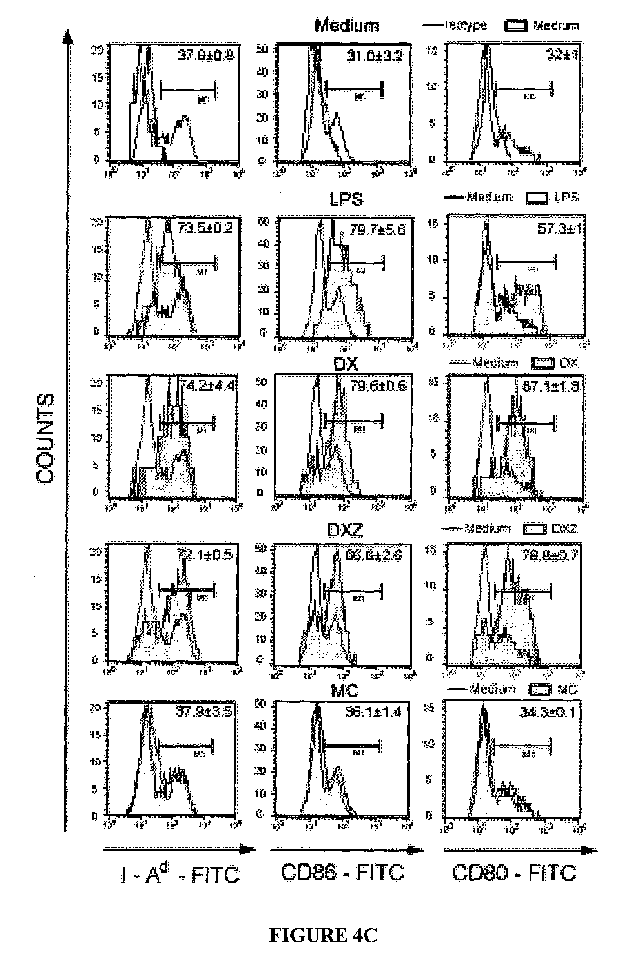

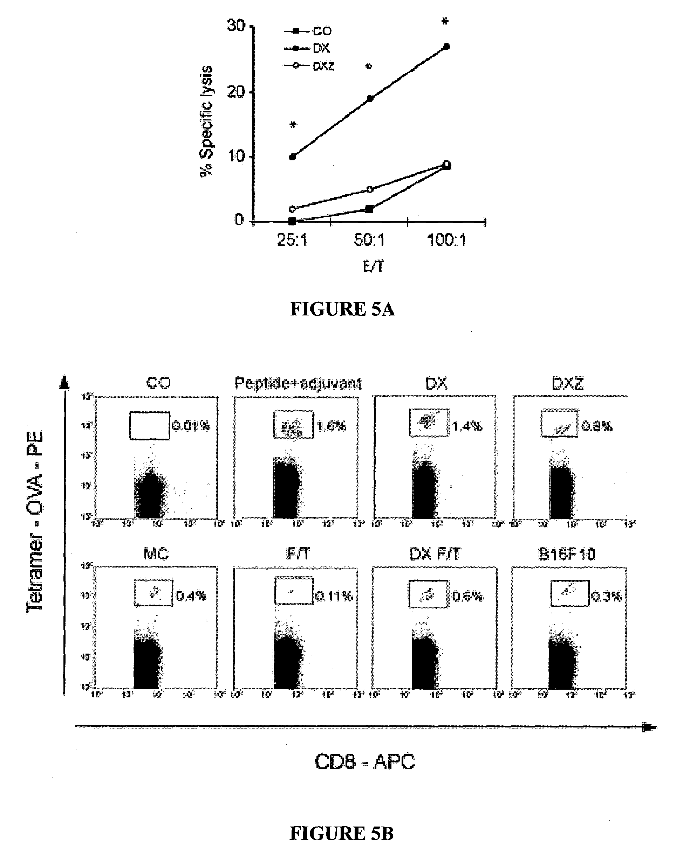

Thus these data underscore a hitherto unsuspected heterogeneity in apoptotic cells with respect to their uptake by dendritic cells³⁴. Importantly, inhibition of caspase activation by Z-VAD-fink or transfected p35 greatly reduced the immunogenic potential of DX-treated cells, an effect that was observed in a variety of different tumor models (Fig. 2-7) and that also affected DC-mediated recognition and phagocytosis (Fig. 4A). However, caspase activation is not sufficient to elicit an immunogenic cell death because MC-treated cells, which do exhibit caspase activation (Fig. 1B), are inefficient anti-tumoral vaccines (Fig. 2). Thus, a specific combination of factors, caspase activation and a yet-to-be defined antigenic property of anthracyclin-treated cells, has to cooperate to elicit an anti-tumor immune response. Disruption of the membrane integrity of DX-treated tumor cells by freeze-thawing is sufficient to abolish their immunogenic property (Fig. 4C, Fig. 5A), meaning that intact apoptotic cells or bodies are required for optimal antigenicity. Irrespective of these mechanistic details, it appears that the particular protocol of apoptosis induction determines the immunogenicity of cell death. Two different subcategories of apoptosis exist, one that is immunogenic and the other that is non-immunogenic.

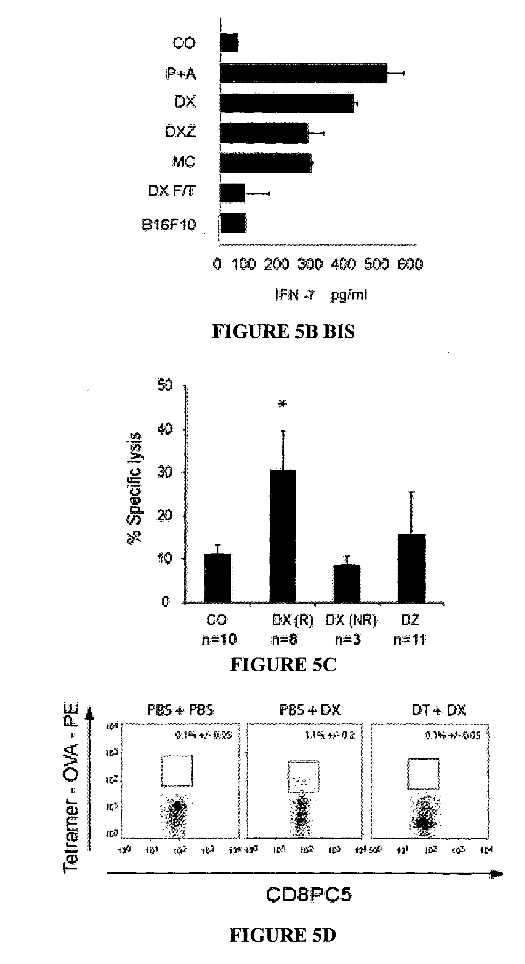

DX-treated, dying tumor cells are efficiently phagocytosed by myeloid and plasmacytoid DC (Fig. 4A,B) and are highly efficient in eliciting antigen-specific cytotoxic T lymphocytes (Fig. 5). Again these properties were largely reduced by simultaneous treatment with the caspase inhibitor Z-VAD-fmk (Fig. 4,5), correlating with a reduced immunogenicity. To our knowledge, this is the first discovery indicating that caspase activation is broadly required for the elicitation of immunogenic tumor cell death. Previous reports have described that caspase activation rather reduces the immunogenic potential of tumor cells. Thus, a PROb clone (REGb) that fails to activate caspases is much more immunogenic than the parental PROb line³⁵. Similarly, a PROb clone manipulated to stably express an anti-sense cytochrome c cDNA undergoes atypical apoptosis without caspase activation, yet has an increased immunogenic potential²⁵. As another example, caspases have been documented to destroy immunodominant epitopes, thus blunting the immune response against lymphoma cells ³⁶. In NOD mice (which are prone to develop autoimmune diabetes), systemic caspase inhibition reduced the priming of T cells by β cells undergoing spontaneous apoptosis ³⁷ and, conversely, local caspase inhibition abolished the tolerogenic effect of β cell apoptosis induced by streptozotocin 33

### Figure legends

### Figure 1: In vitro caracterization of the different cell death modalities induced in CT26 cells.

A. FACS analysis of cells treated in vitro with the indicated agents (DX, doxorubicin; MC, mitomycin C; ZVAD, Z-VAD-fmk; DXZ, doxorubicin plus Z-VAD-fmk, F/T, freeze-thaw), as specified in Materials and Methods. B. Immunoblot analyses of cells treated as in A. Cellular extracts were subjected to the immunodetection of activated caspase-3 (Casp3a), HMGB-1, and HSP-70. C. TUNEL staining (green) of cells counterstained with DAPI (blue). The percentage of TUNEL⁺ cells (X±SEM, n=3) was determined. D. Transmission electron microscopy. Representative microphotographs are shown. Apoptotic cells showing chromatin condensation are labeled with an "A". E. Cells treated with the indicated agents as in A for 19 h were washed and plated to determine the frequency of surviving clones, defining the control value of untreated cells as 100%. Results are representative of three independent experiments.

### Figure 2: Immunogenicity of the different cell death types.

A. Tumor evolution of DX and DXZ treated cells in *nu*/*nu* mice. Cells treated as in Fig. 1 were injected s.c. (3x10⁶/mouse). B. Comparison of the number of tumor-bearing mice between immunocompetent BALB/c and *nu*/*nu* mice. Data from *nu*/*nu* mice are from the same animals as in A, while data from BALB/c mice were obtained on a minimum of 30 animals per group. C. Immunogenic effect of DX-treated tumor cells. BALB/c mice were injected into one flank with live tumor cells and into the opposite flank with DX, DXZ, MC, or F/T-treated cells, and the evolution of tumors was monitored. D. Persistent, specific anti-tumor immunity elicited by DX-treated cells. Animals immunized with DX-treated CT26 cells that remain tumor-free after 120 days (see lower left panel in C) or age-matched control mice were challenged with CT126 cells or the unrelated TSA tumor cell line. E. Effect of the timing of anti-tumor vaccination. CT26 cells treated as in Fig. 1 were injected at the same time as live tumor cells (as in C) or were injected once one week before, twice one and two weeks before or three times, one, two or three weeks before challenge with live tumor cells (n=15 per group). **F.** Replacement of DX by other anthracyclins. Animals were injected with CT26 cells treated daunorubicin (DA) or idarubicin (ID), instead of doxorubicin, or PBS only (CO) and the growth of live CT26 cells simultaneous injected into the opposite flank was monitored as in C. All figures represent 3 to 6 independent experiments. * p < 0.05 vs control (CO).

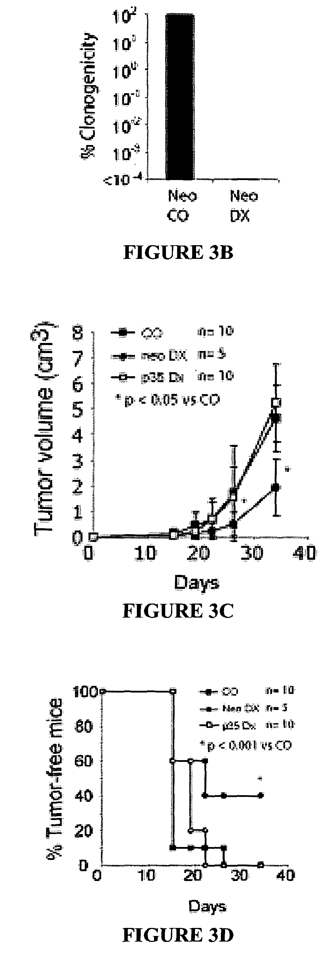

### Figure 3: Inhibition of immunogenicity by the caspase inhibitor p35.

**A.** FACS analysis of CT26 cells transfected with vector only (Neo) or p35, cultured alone or in the presence of DX, as in Fig. 1A. **B.** Clonogenic survival of Neo or p35-transfected cells left untreated (100 % values) or treated with DX. C, D. Evolution of CT26 tumors in animals injected simultaneously with Neo-transfected, DX treated or p35-transfected, DX-treated cells into the opposite flank. Note that only Neo-expressing, DX-treated cells confer tumor immunity (p<0.01).

### Figure 4: Effect of dying tumor cells on dendritic cells.

**A,B.** *In vitro* phagocytosis of the DX, DXZ or MC-treated cells (stained with CMTMR) by spleen DC from Flt3L injected mice. Representative FACS diagrams are depicted in A, and confocal images of dying tumor cells (red) phagocytosed by purified DC cell subset (green) are shown in **B. C.** DC maturation of splenic DC induced by LPS (positive control) and dying or dead or dying CT26 cells. Percentage values in A and C are means of three independent determinations ± SD.

### Figure 5: Characterization of the immune response induced by dying tumor cells.

**A.** CTL response of DX and DXZ-treated cells immunized mice. Splenic T cells from animals vaccinated with PBS only (CO), DX-treated CT26 cells or DXZ-treated CT26 cells were tested for their capacity to lyse syngenic cells pulsed with the immunodominant H2L^{d} restricted CT26-derived peptide SPSYVYHQF. Representative CTL responses from individual mice are shown in the left panel, while mean values are shown on the right. R and NR refer to responders and non-responders, respectively. * p < 0.01 vs control (CO). **B.** Percentages of CD8⁺ lymph node cells expressing TCR that interact with the ovalbumin-derived peptide SIINFEKL presented by H-2K^{b}, five days after challenge with either peptide+adjuvant (P+A), or with DX, DX F/T (DX-treated, then frozen/thawed cells), DXZ, MC and F/T -treated B16-OVA cells (3 experiments). **C.** IFNγ production, as measured after in vitro culture with SIINFEKL in the same experiment. Control values are <50 pg/ml. **D.** Effect of the depletion of DC on the accumulation of specific T cells. Transgenic mice specifically expressing the diphteria toxin (DT) receptor in DC were pretreated with PBS alone or a dose of DT that depletes DC. The mice were then challenged with DX-treated B16-OVA cells into the food pad. Draining lymph nodes were recovered 48 hours later and stained for simultaneous detection of CD8 and OVA peptide-specific T cell receptors. Representative FACS pictograms are shown and values are X±SEM (n=3).

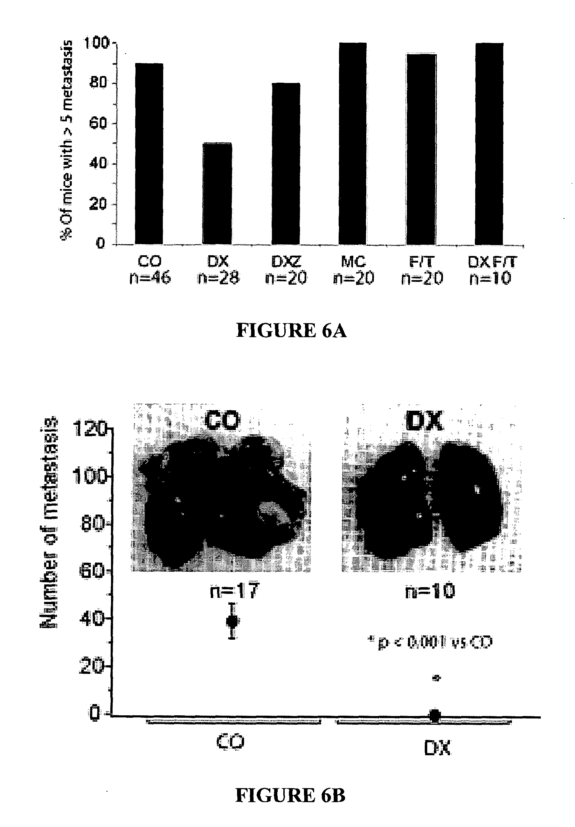

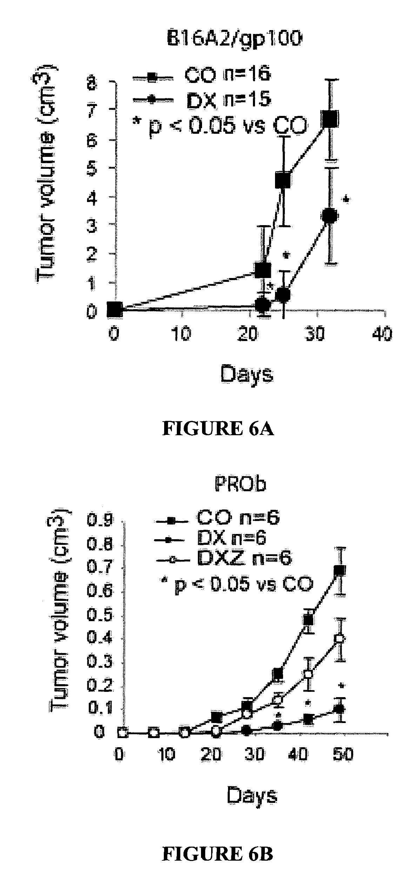

### Figure 6: Anti-tumor vaccination with DX-treated melanoma or colon carcinoma cells in distinct rodent models.

**A.** Number of metastases induced by intravenous injection of CT26 cells in mice injected simultaneously with vehicle only (CO) or dying tumor cells generated as in Fig. 1. Untreated controls developed 46±22 metastastes (X±SD, n=46). **B.** Effect of two immunizations with DX-treated CT26 cells, 14 and 7 days before intravenous injection of live CT 26 cells. All figures are pooled data from 3 independent experiments. * p < 0.001 vs CO. C. DX-treated B16A2 cells were injected twice into mice transgenic for HLA.A2, two weeks and one week before challenge with live B16A2 cells into the opposite flank. * p < 0.05 vs CO. **D.** The protocol of simultaneous s.c. injection of dying (DX or DXZ-treated) tumor cells was applied to BDIX rats challenged with PROb cells. * p < 0.001 vs CO. This experiment has been repeated three times, yielding similar results.

### Figure 7: Ex vivo and in vivo induction of immunogenic cell death.

**A.** Evolution of tumors in mice vaccinated with cells from freshly resected or cryopreserved CT26 tumors, that were left untreated (CO) or treated with DX. **B.** Effect of intratumoral DX injection on the growth of CT26 colon cancer cells inoculated into immunocompetent BALB/c mice. The left panel shows the effect of intratumoral injection of vehicle and the right panel that of DX. While some animals showed a normal tumor growth (n=6) or a simple delay in tumor growth (n=8), other animals exhibited stable disease (n=3) or complete tumor regression (n=7). C. Rechallenge of cured animals (n=7, same mice as in panel B) or age-matched control (CO) mice with live CT26 cells. Similar results were obtained in three independent experiments.

### Example 1 : Immunogenicity of cancer cells treated with anthracyclins

### 1.1 Materials and Methods

**Cell lines and cell death induction.** CT26, PROb, B16F10, B16/F10.9-OVA, and B16F10A2/gp100 (B16F10 transfected with gp100 and HLA A2.1 and selected in 50 µg/ml hygromicin and G418) cells were cultured at 37°C under 5% CO₂ in RPMI 1640 medium supplemented with 10% FCS, penicillin, streptomycin, 1 mM pyruvate and 10 mM Hepes in the presence of doxorubicin (24 h, 25 µM for CT26, 30 µM for PROb, and 2.5 µM for B16F10 and its derivatives), daunorubicin (24h, 5 µM, Pharmacia), idarubicin (24h, 1 µM, Aventis, France), mitomycine C (30 µM, 48 h; Sanofi-Synthelabo, France), and/or zVAD-fink (100 µM, 24 h; Bachem). Necrosis was induced by one freeze/thaw cycle in liquid N₂ and a 37°C water bath. CT26 cells were stably transfected with vector only (Neo) or with a pcDNA3.1 vector encoding p35^{27,28}.

**Cell death assays.** Cells were trypsinized and subjected to cytofluorometric analysis with a FACS Vantage (Becton Dickinson) after staining with 4,6-diamino-2-phenylindole (DAPI, 2.5 µM, 10 min, Molecular Probes) for determination of cell viability, and Annexin V conjugated with fluorescein isothiocyanate (Bender Medsystems) for the assessment of phosphatidylserine exposure⁴¹. TUNEL assays were performed on cells (let to adhere for 1 h in PBS on polylysine-coated slides, O. Kindler GmbH, Germany, fixed with 4% paraformaldehyde for 30 min) using the In situ Cell Death Detection Kit kit by Roche and a Leica IRE2 fluorescence microscope equipped with a Leica DC300F camera. For electron microscopy, cells (fixed for 1 hour at 4°C in 2.5% glutaraldehyde in phosphate buffer (pH 7.4) were washed and fixed again in 2% osmium tetroxide, then embedded in Epon), and ultrathin sections (80 nm) were stained with uranyl acetate and lead citrate and examined with a Leo 902 electron microscope, at 80 kV.

**Immunoblot analyses.** Cells were washed with cold PBS at 4°C and lysed in a buffer containing 50 mM Tris HCl pH 6.8, 10 % glycerol and 2 % SDS. Primary antibodies detecting activated caspase 3 (dilution 1/1000, Cell Signaling Technology), HSP70 (dilution 1/1000, Stratagene) or HMGB-1 (2 µg/ml, Pharmingen), were revealed with the appropriate horseradish peroxidase-labeled secondary antibody (Southern Biotechnologies Associates) and detected by ECL (Pierce). Anti-actin or anti-GAPDH (Chemicon) was used to control equal loading.

**Anti-tumor vaccination and assessment of tumor growth in vivo.** 3×10⁶ treated CT26 cells were inoculated s.c. in 200 µl of PBS into BALB/c six-week-old female mice (Charles River, France), into the lower flank, while 5×10⁵ untreated control cells were inoculated into the contralateral flank⁴². PROb cells (3×10⁶ treated cells and 10⁶ control cells) were injected s.c. into the lower flanks of syngeneic 2-month-old BDIX rats (Charles River), and B16A2 and B16F10 (1.8×10⁵ treated cells and 3×10⁴ control cells) were injected into six-week-old female HHD2 transgenic mice (bred in the IGR animal facility) and C57BL/6 mice (Charles River) respectively. For the tumorigenicity assay, 3x10⁶ treated or untreated CT26 cells were injected s.c. into *nu*/*nu* mice (IGR animal facility). To assess the specificity of the immune response against CT26, we injected either 5x10⁵ or 5x10⁶ of CT26 (for the mice immunize in a standard protocol or vaccination protocol, respectively) or 10⁶ TSA cells. Tumors of the control side were evaluated weekly, using a caliper. Lung metastases were generated by injection of 10⁵ CT26 cells into the tail vein, at the same time when 3x10⁶ treated cells were injected s.c. Mice were injected with Indian ink 20 days later, sacrificed, and lung metastases were counted with a loupe. For vaccination with primary tumours, subcutaneous tumors were dissected and dissociated with collagenase (1.6 mg/ml) and DNAse (350 U/ml) during 20 minutes at 37°C, treated with 20 µM Doxorubicin for 24 hours, and then injected s.c (3x10⁶ cells). Alternatively, the tumors were frozen (in FCS with 10% DMSO) as ~1 mm³ cubes at -80°C and dissociated and treated the same way after thawing. All animals were maintained in specific pathogen-free conditions and all experiments followed the FELASA guidelines.

**Assessment of specific T cell responses.** The CTL response against the immunodominant CT26-specific AH1 H2L^{d}-restricted peptide (SPSYVYHQF, purchased from Neosystem, Strasbourg, France)⁴³, was determined. Splenocytes were isolated from immunized mice 10 days after injection of apoptotic cells and restimulated in vitro for 5 days with or without AH1 (5 µg/ml) in the presence of syngeneic irradiated naive spleen cells. The cytotoxic activity was determined during a classic five-hour in vitro [⁵¹Cr]-release assay, using [⁵¹Cr]-P815 tumor cells loaded with 50 µM AH1 peptide as target cells⁴². The percentage of specific lysis was calculated as 100 x (experimental release - spontaneous release)/(maximal release - spontaneous release). Maximum release was obtained by adding 10% Triton X-405 to target cells and spontaneous release was determined by incubating target cells in medium alone. Female C57BL/6 mice (H-2^{b}, 5-6 weeks old) were injected into both footpads with PBS, 50 µg of OVA peptide (H-2K^{b} restricted, SIINFEKL, aa 257-264, purchased from Eurogentec, Villejuif, France) mixed with incomplete Freund adjuvant (Sigma), or with 10⁶ B16/F10.9-OVA cells. Animals were sacrificed five days after footpad immunization, popliteal and inguinal lymph nodes were removed and processed individually. A single cell suspension was obtained by homogenizing and filtering the organ through a sterile cell strainer (70 µm, Becton Dickinson). For FACS analysis, 2x10⁶ cells were stained at RT with a Tetramer-OVA-PE (Beckman Coulter, UK) and then stained with an anti-CD3-FITC and with an anti-CD8-APC monoclonal antibody (Beckton Dickinson). Cells were analysed using a FACScalibur cytometer (Becton Dickinson) with Cellquest Pro software. Alternatively, 10⁵ cells were cultured in complete culture medium containing 2% of mouse serum, in the presence or absence of 40µg/ml SIINFEKL peptide. Three days later, the supernatants were harvested and IFN-γ secretion was determined by ELISA (OptEIA™ ELISA kit, BD Pharmingen). In ones series of experiments, CD11c-GFP DT K^{b} mice²⁹ were injected i.p. with 100 ng of diphteric toxin (or PBS as a vehicle control), 48 hours before injection of DX-treated B16/F10.9-OVA cells into the food pad.

### 1.2 Results

### A mouse model of vaccination with apoptotic cancer cells.

The colon carcinoma CT26 line was killed by treatment with doxorubicin (DX) or mitomycin C (MC), as determined by FACS analysis (Fig. 1A), although the phenotypic manifestations of cell death differed. Both DX and MC induced caspase activation (Fig. 1B) and chromatin condensation (Fig. 1C, D), but only MC led to TUNEL-detectable DNA fragmentation (Fig. 1C). When combined with DX, the broad spectrum caspase inhibitor Z-VAD-fmk (DXZ) delayed cell death (Fig. 1A), reduced caspase activation (Fig. 1B) and blunted chromatin condensation (Fig. 1D). However, DXZ-treated cells manifested a strongly reduced clonogenic survival as compared to untreated controls, indicating that most of the cells underwent delayed caspase-independent death in vitro (Fig. 1E). Moreover, as compared to untreated controls, DX and DXZ-treated cells generated tumors with a significant delay after inoculation into athymic *nu*/*nu* mice (Fig. 2A). No tumor growth was found when DX, DXZ or MC-treated cells were inoculated into syngenic, immunocompetent BALB/c mice (Fig. 2B). To explore the putative immunogenicity of dead or dying or dying cells, BALB/c mice were inoculated into one flank with live CT26 cells and, simultaneously, received an injection of DX, DXZ, MC-treated or necrotic (frozen-thawed, F/T) cells into the opposite flank. In this therapeutic regime, only DX (but not DXZ, MC or F/T)-treated cells reduced the frequency of tumors developing from live cells by ~45% on day 30 and ~30% on day 120 (Fig. 2C). This degree of protection was obtained without the addition of any adjuvant. It was also independent of the presence of xenogenic antigens (fetal calf serum) in the culture media (not shown). Animals not developing tumors after challenge with DX-treated CT26 cells were protected against a second inoculation of live CT 26 cells, but were not immune against the unrelated TSA tumor (Fig. 2D). Of note, the efficacy of the tumor vaccination could be improved to 80% by one prophylactic injection of DX-treated cells before challenge with live tumor cells. Irrespective of the immunization schedule, DX-treated cells did confer a higher degree of protection than DXZ, MC, or F/T-treated cells (Fig. 2E). Thus, dying tumor cells can elicit an effective anti-tumor immune response.

### Immunogenicity of caspase-dependent, doxorubicin-induced cell death.

DX (Fig. 2C,D) (and other anthracyclins such as daunorubicin and idarubicin, Fig. 2F) induced immunogenic cell death. However, concomitant treatment with the broad-spectrum caspase inhibitor Z-VAD-fmk (DXZ) abolished the immunogenic potential of the treatment with DX (Fig. 2C). No such inhibitory effects were obtained when Z-VAD-fmk was replaced by chemically related caspase inhibitors with a narrow spectrum of action such as Z-DEVD-fmk, Z-IETD-fmk, Ac-LEHD-cmk, Z-VQID-CHO, or Z-VDVAD-CHO, which are specific for caspases 3, 8, 9, 6 and 2, respectively (data not shown). To confirm that caspases (rather than other proteases inhibited by Z-VAD-fink)²⁶ mark the difference between immunogenic (DX) and non-immunogenic (DXZ) death, we stably transfected CT26 cells with the Baculovirus caspase inhibitor p35, a protein that confers highly specific, irreversible caspase inhibition in vitro and in transgenic models^{27,28}. Similarly to Z-VAD-fmk, p35 delayed DX-induced cell death (Fig. 3A), yet did not restore the in vitro clonogenicity of DX treated cells (Fig. 3B). p35-expressing tumor cells failed to form tumors when treated in vitro with DX and inoculated into immunocompetent mice. However, as opposed to vector-only transfected DX-treated controls, p35-transfected DX-treated CT26 cells failed to elicit an anti-tumor immune response (Fig. 3C, D). Animals protected against CT26 wild type tumors also failed to develop tumors after inoculation of live p35-expressing CT26 cells, indicating that p35 did not interfere with the effector arm of the immune system (not shown). Thus, two different protocols of caspase inhibition (pharmacological in the case of Z-VAD-fink; genetic and cell-autonomous in the case of p35) had similar effects on the immunogenicity of DX-treated tumor cells.

### Critical role of dendritic cells (DC) in the immune response elicited by dying tumor cells.

In an attempt to understand why DX-induced cell death is immunogenic, we challenged DC with dead or dying or dying cells and measured their response. DX-treated CT26 cells were phagocytosed by all C11c⁺/IA^{d+} DC subpopulations (CD11b⁺, B220⁺ DC in Fig. 4A and CDBα⁺ DC not shown) present in the spleen, while non-immunogenic (live, DXZ, MC or F/T) CT26 cells were neglected by DC (Fig. 4A,B). In contrast, several categories of dying cells (DX, DXZ, and F/T but not MC) tumor cells possessed the capacity of inducing DC maturation, as indicated by increased expression of the costimulatory molecules CD80 and CD86, as well as induction of MHC class II (Fig. 4C). DX (but not DXZ)-treated CT26 cells elicited a cytotoxic T cell response after in vivo inoculation (Fig. 5A). Similar data were obtained in other tumor-relevant models. Injection of ovalbumin-transfected B16F10 melanoma cells elicited the local recruitment of ovalbumin-specific CD8⁺ T cells (which recognize the K^{b}/SIINFEKL tetramer) into, as well as the production of interferon-γ by cells from the draining lymph node of syngenic C57BL/6 mice, provided that the injected cells were pretreated with DX (Fig. 5B,C). However, this response was blunted when the inoculum had been treated with DXZ, MC or when the DX-treated cells were rendered necrotic by freeze-thawing (Fig. 5B,C). To determine the impact of CD11c⁺ DC on the immune response in vivo, diphteria toxin was injected into transgenic C57BL/6 mice expressing the diphteria toxin receptor specifically in DC (under the control of the CD11c promoter)²⁹. Vehicle-injected control animals recruited CD8⁺ T cells with a TCR specific for the ovalbumin-derived K^{b}/SIINFEKL epitope into the draining lymph node, but DC-depleted animals failed to do so in response to injection of apoptotic ovalbumin-transfected B16F10 cells (Fig. 5D). These data confirm that DX-treated cells are particularly effective in stimulating a cytotoxic immune response and that this response is mediated by DC.

### Doxorubicin-elicited apoptosis is immunogenic in several tumor models.

One single subcutaneous injection of DX-treated CT26 cells conferred a strong protection against the development of pulmonary metastases induced by simultaneous intravenous injection of tumor cells (Fig. 6A). In this system, MC-treated and F/T cells were inefficient; freeze-thawing of DX-treated CT26 cells (DX F/T) annihilated their immunogenic effect; and co-incubation with Z-VAD-fmk led to a partial reduction of the DX effect (Fig. 6A). The anti-metastatic effect was complete when DX-treated CT26 cells were injected twice before intravenous challenge with untreated CT26 cells (Fig. 6B). In a further round of experiments, we determined whether the observed tumor vaccination effects were a general phenomenon. When replacing CT26 cells by B16F10 cells, we found that this melanoma cell line was immunogenic, if DX treated and injected into C57BL/6 mice (not shown). B16F10A2/gp100 melanoma cells present peptides from the human gp100 tumor antigen in the context of HLA class I A2³⁰. Again, in vitro treatment with DX, followed by subcutaneous injection of the cells, was able to induce a significant protection against later challenge with live B16F10A2/gp100 tumor cells, in "humanized" mice expressing an HLA.A2 transgene^{30,31} (Fig. 6C). Thus, DX-treated dying tumor cells turned out to be immunogenic on a human MHC background. Furthermore, inbred BDIX rats inoculated with the syngenic PROb colon carcinoma³² could be protected by injection of DX-treated tumor cells, and this effect was again suppressed by simultaneous pretreatment of the tumor cells with DX and Z-VAD-fmk (Fig. 6D). In conclusion, this protocol of cancer immunization is applicable to a series of distinct tumors, species and MHC backgrounds.

### Ex vivo and in vivo induction of immunogenic apoptosis.

We investigated whether it would be required to immunize animals with dying cells cultured as an established cell line or whether it would be feasible to take advantage of resected tumors (as this would be the case in human cancer patients). CT26 tumors were generated in BALB/c mice and then surgically removed and enzymatically dissociated, optionally after a step of cryostorage in DMSO-containing medium, treated with DX in vitro and used as anti-tumor vaccine (Fig. 7A). This ex vivo removal/in vitro treatment elicited an efficient anti-tumor immune response, if DX was added during the in vitro incubation, although cryostored cells were less efficient than freshly recovered tumor cells. The omission of DX curtailed the efficacy of the vaccine (Fig. 7A). As an alternative to this ex vivo/in vitro protocol of vaccine generation, we injected doxorubicin into palpable (>125 mm³) subcutaneous CT26 tumors established in BALB/c mice. Using this protocol of intratumoral (as opposed to intravenous, not shown) chemotherapy, we achieved stable disease or complete tumor regression in 10 out of 24 (42%) of the animals (Fig. 7B). Rechallenge of animals that manifested a complete therapeutic response with CT26 cells failed to produce tumors (Fig. 7C), indicating that this therapeutic regimen had induced a persistent anti-tumor immunity.

### REFERENCES

1. Zitvogel, L., Casares, N., Pequignot, M., Albert, M.L. & Kroemer, G. The immune response against dying tumor cells. Adv. Immunol. 84, 131-79 (2004).
2. Steinman, R.M. & Mellman, I. Immunotherapy: bewitched, bothered, and bewildered no more. Science 305, 197-200 (2004).
3. Igney, F.H. & Krammer, P.H. Death and anti-death: tumour resistance to apoptosis. Nat Rev Cancer. 2, 277-88 (2002).
4. Kerr, J.F.R., Wyllie, A.H. & Currie, A.R. Apoptosis: a basic biological phenomenon with wide-ranging implications in tissue kinetics. Br. J. Cancer 26, 239-257 (1972).
5. Matzinger, P. The danger model: a renewed sense of self. Science 296, 301-5. (2002).
6. Hanayama, R. et al. Autoimmune disease and impaired uptake of apoptotic cells in MFG-E8-deficient mice. Science 3004, 1147-50. (2004).
7. Asano, K. et al. Masking of phosphatidylserine inhibits apoptotic cell engulfment and induces autoantibody production in mice. J Exp Med. 200, 459-67. (2004).
8. Lauber, K., Blumenthal, S.G., Waibel, M. & Wesselborg, S. Clearance of apoptotic cells: getting rid of the corpses. Mol Cell. 14, 277-87 (14).
9. Fadok, V.A., Bratton, D.L. & Henson, P.M. Phagocyte receptors for apoptotic cells: recognition, uptake, and consequences. J Clin Invest. 108, 957-62 (2001).
10. Kim, S., Elkon, K.B. & Ma, X. Transcriptional suppression of interleukin-12 gene expression following phagocytosis of apoptotic cells. Immunity 21, 643-53 (2004).
11. Hoffmann, P.R. et al. Interaction between Phosphatidylserine and the Phosphatidylserine Receptor Inhibits Immune Responses In Vivo. J Immunol. 174, 1393-404 (2005).
12. Steinman, R.M., Turley, S., Mellman, I. & Inaba, K. The induction of tolerance by dendritic cells that have captured apoptotic cells. J Exp Med. 191, 411-6 (2000).
13. Liu, K. et al. Immune tolerance after delivery of dying cells to dendritic cells in situ. J Exp Med 196, 1091-1097. (2002).
14. Ferguson, T.A. et al. Uptake of apoptotic antigen-coupled cells by lymphoid dendritic cells and cross-priming of CD8+ T cells produce active immune unresponsiveness. J. Immunol. 168, 5589-5595 (2002).
15. Banchereau, J. & Steinman, R.M. Dendritic cells and the control of immunity. Nature 392, 245-252 (1998).
16. Albert, M.L., Sauter, B. & Bhardwaj, N. Dendritic cells acquire antigen from apoptotic cells and induce class I-restricted CTLs. Nature 392, 86-9 (1998).
17. Russo, V. et al. Dendritic cells acquire the MAGE-3 human tumor antigen from apoptotic cells and induce a class I-restricted T cell response. Proc Natl Acad Sci U S A 97, 2185-90 (2000).
18. Iyoda, T. et al. The CD8+ dendritic cell subset selectively endocytoses dying cells in culture and in vivo. J Exp Med 195, 1289-302. (2002).
19. Maranon, C. et al. Dendritic cells cross-present HIV antigens from live as well as apoptotic infected CD4+ T lymphocytes. Proc Natl Acad Sci U S A 101, 6092-7 (2004).
20. Blachere, N.E., Darnell, R.B. & Albert, M.L. Apoptotic cells deliver processed antigen to dendritic cells for cross-presentation. PLoS Biol. in press(2005).
21. Hirschowitz, E.A. et al. Autologous dendritic cell vaccines for non-small-cell lung cancer. J Clin Oncol. 22, 2808-15 (2004).
22. Strome, S.E. et al. Strategies for antigen loading of dendritic cells to enhance the antitumor immune response. Cancer Res 62, 1884-9. (2002).
23. Scheffer, S.R. et al. Apoptotic, but not necrotic, tumor cell vaccines induce a potent immune response in vivo. Int J Cancer. 103, 205-11 (2003).
24. Bartholomae, W.C. et al. T cell immunity induced by live, necrotic, and apoptotic tumor cells. J Immunol. 173, 1012-22 (2004).
25. Schmitt, E. et al. Increased immunogenicity of colon cancer cells by selective depletion of cytochrome c. Cancer Res 64, 2705-11 (2004).
26. Rozman-Pungercar, J. et al. Inhibition of papain-like cysteine proteases and legumain by caspase-specific inhibitors: when reaction mechanism is more important than specificity. Cell Death Differ. 10, 881-8 (2003).
27. Stennicke, H.R., Ryan, C.A. & Salvesen, G.S. Reprieval from execution: the molecular basis of caspase inhibition. Trends Biochem Sci. 27, 94-101 (2002).
28. Date, T. et al. Myocardial expression of baculoviral p35 alleviates doxorubicin-induced cardiomyopathy in rats. Hum Gene Ther. 14, 947-57 (2003).
29. Jung, S. et al. In vivo depletion of CD11c(+) dendritic cells abrogates priming of CD8(+) T cells by exogenous cell-associated antigens. Immunity 17, 211-20 (2002).
30. Mullins, D.W., Bullock, T.N., Colella, T.A., Robila, V.V. & Engelhard, V.H. Immune responses to the HLA-A*0201-restricted epitopes of tyrosinase and glycoprotein 100 enable control of melanoma outgrowth in HLA-A*0201-transgenic mice. J Immunol. 167, 4853-60 (2001).
31. Shirai, M. et al. CTL responses of HLA-A2. 1-transgenic mice specific for hepatitis C viral peptides predict epitopes for CTL of humans carrying HLA-A2.1. J. Immunol. 154, 2733-42 (1995).
32. Caignard, A., Pelletier, H. & Martin, F. pecificity of the immune response leading to protection or enhancement by regressive and progressive variants of a rat colon carcinoma. Int J Cancer. 42, 883-6 (1988).
33. Hugues, S. et al. Tolerance to islet antigens and prevention from diabetes induced by limited apoptosis of pancreatic beta cells. Immunity 16, 169-81 (2002).
34. Demaria, S. et al. Select forms of tumor cell apoptosis induce dendritic cell maturation. J Leukoc Biol. 77, 361-368. (2005).
35. Larmonier, N. et al. An atypical caspase-independent death pathway for an immunogenic cancer cell line. Oncogene 21, 6091-100 (2002).
36. Castiglioni, P. et al. Apoptosis-dependent subversion of the T-lymphocyte epitope hierarchy in lymphoma cells. Cancer Res. 62, 1116-22 (2002).
37. Turley, S., Poirot, L., Hattori, M., Benoist, C. & Mathis, D. Physiological beta cell death triggers priming of self-reactive T cells by dendritic cells in a type-1 diabetes model. J Exp Med. 198, 1527-37 (2003).
38. Tamm, I. et al. Expression and prognostic significance of IAP-family genes in human cancers and myeloid leukemias. Clin Cancer Res. 6, 1796-803 (2000).
39. Ferreira, C.G. et al. Expression of X-linked inhibitor of apoptosis as a novel prognostic marker in radically resected non-small cell lung cancer patients. Clin Cancer Res. 7, 2468-74 (2001).
40. Muris, J.J. et al. mmuno-histochemical profiling of caspase signaling pathways predicts clinical response to chemotherapy in primary nodal diffuse large B-cell lymphomas. Bloode [Epub ahead of print](2005).
41. Castedo, M. et al. Sequential involvement of Cdk1, mTOR and p53 in apoptosis induced by the human immunodeficiency virus-1 envelope. EMBO J. 21, 4070-4080 (2002).
42. Casares, N. et al. Immunization with a tumor-associated CTL epitope plus a tumor-related or unrelated Th1 helper peptide elicits protective CTL immunity. Eur. J. Immunol. 31, 1780-1789 (2001).
43. Huang, A.V. et al. The immunodominant major histocompatibility complex class I-restricted antigen of a murine colon tumor derives from an endogenous retroviral gene product. Proc Natl Acad Sci U S A 93, 9730-9735 (1996).

## Claims

1. A process of manufacturing an immunogenic composition comprising the steps consisting of :
a) suspending or plating tumor cells or infected cells in a culture media,
b) treating said cells with at least one antineoplastic compound selected from anthracyclins and vinorelbine in a sufficient amount to induce cellular apoptosis, wherein said apoptosis induced by anthracyclins produces immunogenic dead or dying cells, and
c) recovering immunogenic dead or dying cells and fragments thereof from the culture media.

2. The process according to claim 2, wherein the tumor cells in step a) are the tumor cells of a human subject allowing the manufacture of a tailored immunogenic composition from one patient's own cancer cells to treat said patient (autologous treatment).

3. The process according to claim 2, wherein it is performed using established cancer cell lines or freshly harvested cancer cells from one or several patients (allogenic treatment).

4. The process according to one of claims 1 to 3, wherein tumor cells are solid tumor cells, such as carcinomas, melanomas and sarcomas.

5. The process according to one of claims 1 to 3, wherein tumor cells are blood cancer cells, such as lymphomas and leukemias.

6. The process according to claim 1, wherein cells in step a) are infected cells including cells infected with a virus such as HIV or Hepatitis C virus.

7. The process according to one of claims 1 to 6, allowing to target specifically in vivo appropriate antigen presenting cells such as plasmacytoid dendritic cells or myeloid dendritic cells for optimal CTL priming or T cell activation.

8. The process according to one of claims 1 to 6, allowing ex vivo pulsing or loading of tumor antigens on to ex vivo propagated allogeneic or autologous dendritic cells for further adoptive transfer in vivo in tumor bearing patients or infected patients.

9. The process according to one of claims 1 to 8, further comprising one or several of the following steps : purification, elutriation, ultracentrifugation and loading into myeloid or plasmacytoid dendritic cells of immunogenic dead or dying cells and fragments thereof.

10. The process according to one of claims 1 to 9, wherein anthracyclin compounds are anthracyclin glycosides selected from doxorubicin, 4'-epi-doxorubicin, 3',4'-diepidaunorubicin and 3',4'-diepidoxorubicin, 4'-desoxy-doxorubicin, daunorubicin, 5-iminodaunorubicin, and 4-demethoxy-daunorubicin, especially a compound having the formula : 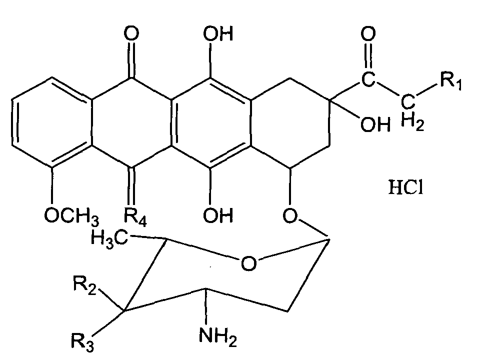 wherein and R1, R2, R3 are independently selected from H, OH, a halogen, a C1-C4 alkyl, alkenyl, or alkoxy group, such as
| | **R1** | **R2** | **R3** | **R4** |
|---|---|---|---|---|
| Doxorubicin | OH | H | OH | O |
| Daunorubicin | H | H | OH | O |
| Epirubicin | OH | OH | H | O |
| 5-iminodaunorubicin | H | H | OH | NH |

11. The process according to one of claims 1 to 10, wherein cancer cells are treated with doxorubicin.

12. The use of the process according to one of claims 1 to 11 for the manufacture of a medicament or a vaccine for preventing or treating cancer and infectious diseases.

13. A composition comprising immunogenic tumor dead or dying cells or infected dead or dying cells and fragments thereof obtainable by the process as defined in one of claims 1 to 11.

14. A composition comprising immunogenic dead or dying cancer cells or infected cells and fragments or fractions thereof obtained by treatment with anthracyclins.

15. The composition according to claim 13 or 14 comprising anthracyclin glycosides treated cancer cells or infected cells and fragments thereof which is in the form of a dead or dying cellular extract or fraction thereof or of a purified cellular lysate.

16. The composition according to one of claims 13 to 15 further comprising at least one of the following : a pharmaceutical acceptable carrier, one or several adjuvant(s) and/or active ingredient(s), in particular agents which are cytotoxic for tumors, such as a compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin, caspase activators such as inhibitors of LAP proteins, or all kinds of adjuvants including cytokines and interleukines, such as interferon-alpha, granulocyte-macrophage colony stimulating factor, soluble CD40 ligand, Flt3 ligand and/or interleukin-2, ligands of Toll-like receptors, neutralizing antibodies such as anti-CD25, anti-CTLA4, and stimulating antibodies such as anti-CD40, tyrosine kinase inhibitors such as c-kit inhibitors, cyclophosphamide in an amount suitable for immunostimulation, or small interfering RNAs inhibiting immunosupressive genes.

17. The composition according to one of claims 13 to 16 which is suitable for intravenous, intradermic, intramuscular or subcutaneous injection, as well as oral or nasal administration.

18. An extract of immunogenic dead or dying infected cells or cancer cells and fragments thereof obtainable by the process as defined in one of claims 1 to 11, such as a dead or dying cellular extract or fraction thereof or of a purified cellular lysate.

19. The use of an extract of immunogenic dead or dying infected cells or cancer cells and fragments thereof according to claim 18 for preparing a medicament for preventing or treating cancer.

20. A method of preventing or treating infection or cancer comprising administering a composition or extract of immunogenic dead or dying infected cells or cancer cells obtainable by a process comprising :
a) obtaining fresh infected cells or cancer cells from a patient,
b) suspending or plating said cells a) in a culture media,
c) treating cells of step b) with at least one antineoplastic compound selected from anthracyclins in a sufficient amount to induce cellular apoptosis, wherein said apoptosis induced by anthracyclins leads to immunogenic tumor cell death,
d) recovering immunogenic dead or dying cells and fragments thereof from the culture media, and
e) administering the immunogenic dead or dying cells and fragments thereof, optionally purified, to said patient.

21. A method according to claim 20, further comprising extraction, filtration, purification, and/or sterilization protocols to obtain an administrable composition.

22. A method according to claim 20, further comprising lyophilization and resuspension in a solution or buffer such as an appropriate sterile saline solution, allowing one or several administrations over time.

23. A method according to one of claims 20 to 22, which is tailored to one particular patient when the immunogenic dead or dying cells and fragments thereof composition is prepared from the cancer cells of said patient.

24. A method according to one of claims 20 to 22, which is practiced using one or several cell lines corresponding to one particular cancer type.

25. A method of preventing or treating cancer in a patient in need of such treatment comprising administering a composition or extract of immunogenic dead or dying cancer cells obtainable by a process comprising :
a) suspending or plating in a culture media cancer cells from cancer cell lines corresponding to the cancer type afflicting said patient,
b) treating cells of step a) with at least one antineoplastic compound selected from anthracyclins in a sufficient amount to induce cellular apoptosis, wherein said apoptosis induced by anthracyclins leads to immunogenic tumor cell death,
d) recovering immunogenic dead or dying cells and fragments thereof from the culture media, and
e) administering the immunogenic dead or dying cells and fragments thereof, optionally purified, to said patient.

26. A method according to claim 25, wherein the cells of step a) derive from established appropriate cell lines or from harvested cancer cells from one or several patients afflicted with a particular cancer including solid tumors such as carcinomas and melanomas and blood cancer such as lymphomas.

27. A method of preventing or treating cancer comprising injected directly in situ into the cancer mass or at the vicinity of localized cancer cells of the patient a compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin in an amount sufficient to induce immunogenic cancer cell death.

28. Use of a compound selected from anthracyclin glycosides, preferably doxorubicin, 4'-epi-doxorubicin, 3',4'-diepidaunorubicin, 3',4'-diepidoxorubicin, 4'-desoxy-doxorubicin, daunorubicin, 5-iminodaunorubicin or 4-demethoxy-daunorubicin for preparing a medicament suitable to direct injection in situ into the cancer mass or at the vicinity of localized cancer cells of the patient for inducing immunogenic cancer cell death.

29. The use according to claim 28 for preparing a medicament for the treatment of solid tumors such as carcinomas, melanomas and sarcomas.
